(19) Europäisches Patentamt
European Patent Office
Office européen des brevets

(11) EP 4 744 656 A1

(12) EUROPEAN PATENT APPLICATION
published in accordance with Art. 153(4) EPC

(43) Date of publication:
20.05.2026 Bulletin 2026/21

(21) Application number: 24838801.9

(22) Date of filing: 09.07.2024

(51) International Patent Classification (IPC):
A61K 31/19 (2006.01)     A61K 31/185 (2006.01)
A61P 17/02 (2006.01)     A61P 19/04 (2006.01)
A61P 41/00 (2006.01)

(52) Cooperative Patent Classification (CPC):
A61K 31/185; A61K 31/19; A61K 31/295;
A61K 31/315; A61P 17/02; A61P 19/00;
A61P 19/04; A61P 41/00

(86) International application number:
PCT/CN2024/104438

(87) International publication number:
WO 2025/011549 (16.01.2025 Gazette 2025/03)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 10.07.2023 CN 202310840152

(71) Applicant: Changchun Sinobiomaterials Co., Ltd.
Changchun, Jilin 130000 (CN)

(72) Inventors:
• WANG, Jinyue
  Changchun, Jilin 130000 (CN)
• ZHUANG, Xiuli
  Changchun, Jilin 130000 (CN)
• ZHANG, Tianhui
  Changchun, Jilin 130000 (CN)

(74) Representative: Colombo, Stefano Paolo et al
Marchi & Partners S.r.l.
Via Vittor Pisani, 13
20124 Milano (IT)

Remarks:
The complete document including Reference
Table(s) and the Sequence Listing(s) can be
downloaded from the EPO website

(54) **USE OF DIVALENT METAL LACTATE IN THE PREPARATION OF PRODUCTS FOR PROMOTING TISSUE GROWTH AND REPAIR**

(57) Provided is use of a divalent metal lactate in the preparation of products for promoting the regeneration, growth, healing, and injury repair of tissues, belonging to the technical field of biomedicines. Preferably, the divalent metal lactate comprises one or more of calcium lactate, magnesium lactate, zinc lactate, and ferrous lactate. Further provided is a novel treatment method for repairing tissue injuries, and preventing and treating fibrosis of tissues and/or scar tissue generation, the treatment method having broad prospects of applications in the treatment and repair of injuries in tissues such as skin, connective tissues, tendons, and fasciae.

EP 4 744 656 A1

**Description**

**CROSS-REFERENCE TO RELATED APPLICATIONS**

The application is based on and claims priority to CN application number 202310840152.1 filed on July 10, 2023, the entire contents of which are hereby incorporated by reference in the present application.

**TECHNICAL FIELD**

The present invention belongs to the technical field of biomedicines, specifically relates to use of a divalent metal lactate in the preparation of a product for promoting the regeneration, growth, healing, and injury repair of a tissue.

**BACKGROUND**

Mammals have evolved a rapid response that triggers a healing response of the body when injured to prevent life-threatening bleeding and infection, so as to ensure survival. The wound healing process goes through different stages, some of which occur sequentially, while others occur simultaneously. However, all stages work in close coordination at the site of injured tissues to regenerate tissues with normal function. The tissue regeneration, growth, healing, and injury repair events at the site of injuries sequentially involve coagulation, inflammation, tissue deposition (migration and proliferation), and the final tissue reconstruction.

When tissue is injured, blood is first released from the injured blood vessels, thus leading to the formation of fibrin fiber meshes into which platelets are embedded. These fibrin fiber meshes can serve as a scaffold for recruited cells to move toward and throughout them. Activated platelets degranulate and release chemokines, including cytokines and growth factors such as transforming growth factor-β1 (TGF-β1), thus leading to the recruitment of fibroblasts and keratinocytes. A few days after the injury, fibroblasts begin to replace the injured tissue by depositing new collagen matrix. The collagen fibers gradually increase in thickness and line up along the wound. In normal scar formation, collagen fibers are generally arranged parallel to the epidermis. Through myofibroblasts, this newly formed granulation tissue eventually builds up and contracts into a denser structure.

Scars typically form as a result of the normal progression of the wound healing response and consist of connective tissues deposited during the healing process. Most scars show some degree of abnormal structures (as can be seen in skin scars) and a certain amount of abnormal connective tissues (as can be seen in central nervous system scars). However, if the production of normal tissue is less than optimal wound healing, excessive deposition of scar tissues is followed, leading to formation of keloids and hypertrophic scars, also known as fibrosis. Scar formation is generally harmful, as it would affect the appearance of tissues such as skin, ligaments, tendons, or fasciae, and more seriously, it would also result in impaired function of these tissues.

The goals of clinically treating and repairing scars are to restore functions, alleviate symptoms, improve appearance, and prevent recurrence. Methods for clinically treating scars include: surgery, medication, physical abrasion, pressure therapy, radiation therapy, chemical peels, cryotherapy, radio frequency microneedling treatment, ion beam therapy, laser, and so on. It can be seen that most of the treatment methods currently used in clinical practice are invasive and need to be completed in a hospital, therefore having poor inconvenience and a relatively heavy financial burden on patients. In terms of medication, the types of drugs available are limited, and the therapeutic effect is not good. Therefore, there is a continuous and urgent demand for safe, effective, inexpensive, and easy-to-administered products in clinical practice that can be used to promote tissue regeneration, growth, healing, and injury repair.

**SUMMARY OF THE INVENTION**

In previous studies, the inventors discovered that during the degradation of poly-L-lactic acid (PLLA), the molecular structure of PLLA is gradually destroyed and slowly hydrolyzed into lactic acid. Lactic acid can induce human fibroblasts to increase collagen production, leading to an increase in collagen fibers in the dermis and producing a filling and repairing effect. Based on this, the inventors have further discovered that PLLA and lactic acid and their related lactate compounds have beneficial effects on repairing cartilage, connective tissue, tendons, fascia, nerves and other tissues (see CN202210028046.9). The present invention is a continuation of the previous work. The present invention has discovered that the divalent metal lactate can not only promote tissue regeneration, growth, and healing, but also prevent and treat tissue fibrosis and/or scar tissue generation; therefore, said divalent metal lactate has broad prospects of applications in the treatment and repair of injuries in tissues such as skin, connective tissues, tendons, and fasciae.

Therefore, the present invention provides new uses of divalent metal lactates. During their research, the inventors unexpectedly discovered that the divalent metal lactate can effectively regulate and promote tissue regeneration, growth, healing, and injury repair, and based on this discovery, they conducted in-depth research and completed the present invention.

To achieve the objectives of the present invention, the following technical solutions are adopted:

In a first aspect, the present invention provides the use of a divalent metal lactate in the preparation of a product for promoting the regeneration, growth, healing (such as wound healing) and/or injury repair of a tissue, wherein the tissue is selected from collagen-rich tissues.

In a second aspect, the present invention provides the use of a divalent metal lactate in the preparation of a product that prevents or treats an injury of a tissue, wherein the tissue is selected from collagen-rich tissues.

In a third aspect, the present invention provides the use of a divalent metal lactate in the preparation of a product that prevents or treats the fibrosis and scar generation of a tissue, wherein the tissue is selected from collagen-rich tissues.

As an optional manner, in the above aspects, the tissue is selected from at least one of skin, ligaments, tendons (such as achilles tendon), or fasciae.

As an optional manner, in the above aspects, the divalent metal lactate comprises one or more of calcium lactate, magnesium lactate, zinc lactate, and ferrous lactate.

As an optional manner, in the above aspects, the divalent metal lactate is selected from zinc lactate or a composition of zinc lactate and magnesium lactate.

Preferably, in the composition, the usage ratio of the zinc lactate to the magnesium lactate is 1:10-10:1, preferably 1:1, based on the amount of substance.

More preferably, in the composition, the usage ratio of the zinc lactate to the magnesium lactate is 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, or 10:1, based on the amount of substance.

As an optional manner, in the above aspects, the product is selected from one or more of a drug, a kit, a health product, and a medical device.

Preferably, the concentration of the divalent metal lactate in the product is 5-250 mmol/L.

Preferably, the concentration of the divalent metal lactate in the product is 15-250 mmol/L.

More preferably, the content of the divalent metal lactate in the skin, ligaments, tendons or fasciae is 30-150 mmol/L.

Preferably, when the product is selected from a drug or a health product, the concentration of the divalent metal lactate in a unit dose of the product is 5-250 mmol/L, preferably 30-150 mmol/L, more preferably 50 mmol/L.

more preferably, when the divalent metal lactate is a composition of zinc lactate and magnesium lactate, the sum of the concentrations of the zinc lactate and the magnesium lactate in a unit dose of the product is 5-250 mmol/L, preferably 30-150 mmol/L, more preferably 50 mmol/L; preferably, the concentration of the zinc lactate and the concentration of the magnesium lactate are the same in the composition.

As an optional manner, in the above aspects, the medical device comprises one or more of medical adhesive tape, bandage, gauze, plaster, sponge, and medical sutures.

As an optional manner, in the above aspects, in the drug, the divalent metal lactate is an active ingredient, and the drug further comprises a pharmaceutically acceptable carrier or excipient.

Preferably, in the drug, divalent metal lactate is the only active ingredient.

In the drug, the dosage form of the drug comprises an injection or a topical preparation.

Preferably, the topical preparation comprises one or more of ointments, creams, patches, sprays, solutions, and lotions.

Preferably, the product is administered by one or more of the following routes: intravenous injection, in situ injection, intramuscular injection, subcutaneous injection, oral administration, or topical application.

In addition, those skilled in the art will know that the manner of use of the product, as well as the dosage and volume of application, are related to the age, physical condition and disease of the subject, and can be determined by a clinician as appropriate. The term "unit dose" refers to the amount of drug that produces a certain therapeutic response after a single administration. For example, when the preparation is in a form of liquid preparation, "unit dose" refers to the amount of drug that produces a certain therapeutic response after a single injection or topical application.

Compared with the prior art, the present invention has the following advantages:

The present invention provides a new treatment method that is safe, effective, inexpensive, and easy to be administered in terms of repairing tissue injuries and preventing and treating tissue fibrosis and/or scar tissue generation. In particular, the present invention has discovered the use of divalent metal lactates, comprising one or more of calcium lactate, magnesium lactate, zinc lactate, and ferrous lactate, in the preparation of products for promoting regeneration, growth, healing, and/or injury repair of tissues. Therefore, the present invention has broad prospects of applications in the treatment and repair of injuries in tissues such as skin, connective tissues, tendons, fasciae, etc.

**BRIEF DESCRIPTION OF THE DRAWINGS**

FIG. 1: Achilles tendon staining images of the experimental side (50 mmol/L calcium lactate aqueous solution) and the control side (0.9% sodium chloride injection solution).

FIG. 2: Achilles tendon staining images of the experimental side (50 mmol/L magnesium lactate aqueous solution) and the control side (0.9% sodium chloride injection solution).

FIG. 3: Achilles tendon staining images of the experimental side (50 mmol/L zinc lactate aqueous solution) and the control side (0.9% sodium chloride injection solution).

## DETAILED DESCRIPTION OF THE INVENTION

The following provides a further explanation of the present invention by referring to specific examples. It should be understood that the specific examples described herein are for illustrative purposes only and are not intended to limit the scope of the invention.

If the specific techniques or conditions are not indicated in the examples, the techniques or conditions described in the literature in the art or the product instructions shall be followed. The reagents and instruments used without indicating the manufacturer are all conventional products that can be purchased through normal channels.

The experimental methods in the following examples are all conventional methods, unless otherwise specified. Unless otherwise specified, all test materials used in the following examples are commercially available products.

**Example 1: Effects of Divalent Metal Lactates on Tendon Growth in Experimental Animals**

See CN202210028046.9 for the main experimental methods and procedures. The details are as follows:

1. Experimental Design:

Experimental animals: SD rats weighing 200-220 g (both male and female, 1:1 ratio) were selected and divided into three groups with 10 rats in each group.

Experimental site: the right achilles tendons of rats were selected as the tendons.

The experimental grouping information are shown in Table 1 below.

**Table 1: Experimental grouping and treatment**

| Group | Components | Concentration | Injection volume |
|---|---|---|---|
| Calcium lactate group | Calcium lactate aqueous solution | 50 mmol/L | 0.1 mL |
| Magnesium lactate group | Magnesium lactate aqueous solution | 50 mmol/L | 0.1 mL |
| Zinc lactate group | Zinc lactate aqueous solution | 50 mmol/L | 0.1 mL |

Experimental procedures: the anatomical position of the right achilles tendon of the rat was determined. The skin on the dorsal side of the distal tibia was disinfected with an alcohol tampon. Using a 26G needle and a 1 mL sterile syringe, 0.1 mL of 50 mmol/L lactate solution was injected into the area around the achilles tendon. The right side was designated as the experimental side (0.1 mL of different types of divalent metal lactate solutions were injected), and the left side was designated as the control side (0.1 mL of 0.9% sodium chloride injection solution was injected).

During the first 1-7 days of the experiment, the experimental solution of the corresponding group was injected at a single point on the experimental side every day, and the same volume of sodium chloride injection solution was injected at a single point on the control side. Three rats from each group were sacrificed on day 3, day 7 and day 14 after surgery. The experimental side and the control side of each rat were picked for fixation and histopathological examination.

2. Determination Methods:

1) The achilles tendon tissue to be observed was excised. After the achilles tendon tissue was fixed, Picro Sirius Red stain was performed to observe the effect of the injection solution generated on the achilles tendon tissue.

2) The experimental side and the control side were randomly picked for sectioning, the tissue diameters were measured under a 100x optical microscope, and the average values of the experimental side and the control side were calculated using the following formula:

Average value = (3 measurements of animal #1 + 3 measurements of animal #2 + 3 measurements of animal #3) / 9

By comparing the diameters on both sides, the effects of the divalent metal lactates on achilles tendon tissues were analyzed by using the calculation results in SPSS 21.

3. Experimental Results:

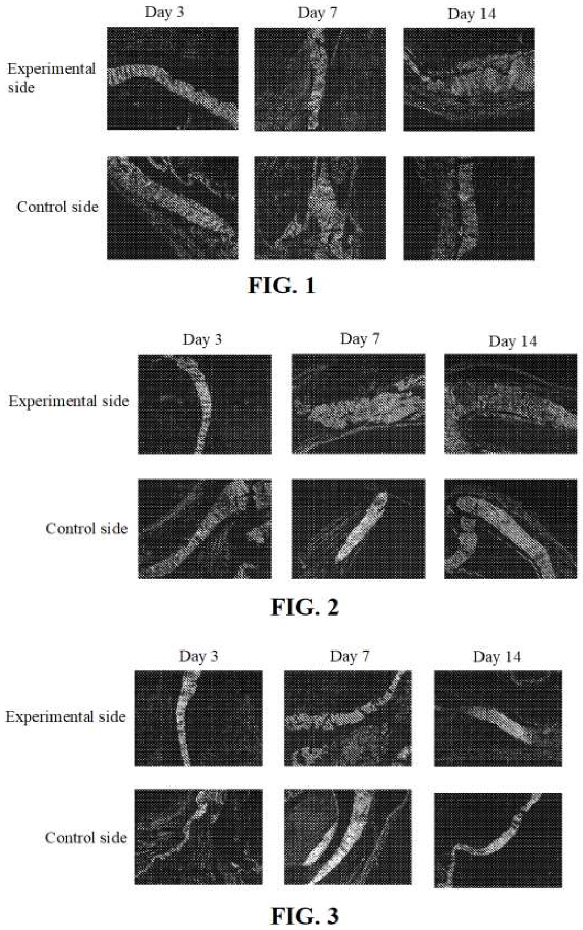

The experimental results are shown in FIG. 1, FIG. 2 and FIG. 3 and Table 2 below. After injection of 50 mmol/L divalent metal lactate solutions, Significant thickening of the achilles tendon tissue on the experimental side to varying degrees can be seen in the experimental animals in each group. As can be seen from the figures, significant thickening of the experimental side and an increase in the diameter of the achilles tendon can be seen in the zinc lactate solution group on day 3, day 7, and day 21. The promoting effect of the zinc lactate solution group on achilles tendon growth was superior to that of the calcium lactate solution and the magnesium lactate solution.

The measurement values of the achilles tendon tissues under a 100x optical microscope are shown in Table 2. Compared with the control side, there was a statistically significant difference among the achilles tendon diameters on the experiment side on day 7 and day 14 in the zinc lactate treatment group ($P < 0.05$) with significant achilles tendon diameter increases observed, demonstrating a significant promoting effect of the zinc lactate treatment on achilles tendon growth. Compared with the control side, there was a statistically significant difference among the achilles tendon diameters on the experiment side on day 7 in the calcium lactate treatment group ($P < 0.05$) with achilles tendon diameter increases observed, demonstrating a promoting effect of the calcium lactate treatment on achilles tendon growth. Compared with the control side, there were increases of the achilles tendon diameters on the experiment side on day 7 and day 14 in the magnesium lactate treatment group, but the promoting effect of the magnesium lactate treatment on achilles tendon growth was not so obvious.

As can be seen from these experimental results, the divalent metal lactates have significant promoting effects on the regeneration of animal tendon tissues, especially the zinc lactate treatment which exhibited an extremely significant promoting effect.

**Table 2: Measurement values of the experimental side and the control side**

|  | Time node | Group | Mean ($\mu$m) | Standard deviation | *P*-value |
|---|---|---|---|---|---|
| Calcium lactate group | Day 7 | Experimental group | 970.09 | 47.55 | 0.041 |
|  |  | Control group | 681.75 | 207.65 |  |
|  | Day 14 | Experimental group | 578.19 | 137.55 |  |
|  |  | Control group | 509.73 | 66.21 | 0.298 |
| Magnesium lactate group | Day 7 | Experimental group | 877.11 | 213.79 | 0.071 |
|  |  | Control group | 659.69 | 154.67 |  |
|  | Day 14 | Experimental group | 547.75 | 95.77 | 0.202 |
|  |  | Control group | 462.17 | 94.43 |  |
| Zinc lactate group | Day 7 | Experimental group | 970.86 | 216.66 | 0.027 |
|  |  | Control group | 617.52 | 57.90 |  |
|  | Day 14 | Experimental group | 832.20 | 126.22 | 0.003 |
|  |  | Control group | 514.63 | 102.30 |  |

**Example 2: Effects of Divalent Metal Lactates on the Treatment of Ligament Injuries in Experimental Animals**

1. Establishment of Anterior Cruciate Ligament Injury Model and Experimental Grouping

A total of forty 3-month-old New Zealand white rabbits (both male and female, 1:1 ratio) weighing 2.5 kg $\pm$ 200 g are kept in a temperature-controlled room (22 $\pm$ 2°C). Before the experiment, 3% (1 mL/kg) pentobarbital sodium was injected via the rabbits' ear vein. After the rabbits were anesthetized, they were fixed on the operation table. The hair in the knee joint surgical area was shaved, and a 2 cm longitudinal incision was made along the medial side of the patella. After the skin was cut open, the fasciae and muscles were carefully separated. After fully exposing the joint cavity, a row of 3 eyeholes were punctured at the middle-to-upper 1/3 of the distal femur of the anterior cruciate ligament, perpendicular to the long axis of the ligament, using the oblique section of a 20 mL needle, so as to simulate an shear force injury to the anterior cruciate ligament in clinical practice. After the model establishment was completed, the surgical area was closed, and sutures and pressure bandages were applied. After the surgery, the animals were randomly divided into 4 groups of 10 each.

After the model was established, 0.25 mL of physiological saline was injected around the injured ligament for the control group; and 0.25 mL of the corresponding lactate aqueous solution was injected around the injured ligament for the experimental group, 0.25 mL each time. Once a day for 4 consecutive weeks.

**Table 3: Experimental grouping and treatments**

| Group | Rabbit number | Dosage | Volume of administration |
|---|---|---|---|
| Control group | 10 | The corresponding volume of normal saline | 0.25 mL |
| Magnesium lactate group | 10 | 50 mmol/L | 0.25 mL |
| Magnesium lactate + zinc lactate group | 10 | 25 mmol/L + 25 mmol/L | 0.25 mL |
| Zinc lactate group | 10 | 50 mmol/L | 0.25 mL |

The limb motor function after the anterior cruciate ligament injury was evaluated by comparing the vertical take-off heights of rabbits in different experimental groups. The results found that the vertical take-off heights of the animals in the experimental groups were significantly greater than those in the control group ($P < 0.05$). The vertical take-off heights of the animals in the magnesium lactate and zinc lactate combination group were greater than those in the magnesium lactate group ($P < 0.05$).

2. Tissue Sample Acquisition

Rabbits were euthanized by injection of an excessive amount of 3% pentobarbital sodium via the ear vein. The surgical area was cleaned by shaving the hair and disinfected. The skin was cut open along the patella, and the fasciae and muscles were carefully separated. The incision was cleaned with PBS solution. The entire anterior cruciate ligament was cut off at both ends close to the tibia and femur for total RNA extraction.

3. Real-time quantitative PCR

3.1 Extraction of total RNA

The ligament was taken out, with addition of 0.5 mL Trizol, ground thoroughly, and centrifuged to collect the supernatant. 0.1 mL of chloroform was added, the mixture was centrifuged, and the supernatant was collected. 0.8 mL of isopropanol was added, the mixture was centrifuged, and the supernatant was discarded. 1 mL of 75% ethanol was added, the mixture was centrifuged, and the supernatant was discarded. 20 $\mu$L of DEPC-treated water was added to dissolve the RNA. The concentration and purity of the total RNA solution were determined using a nucleic acid protein detector.

3.2 Real-time quantitative PCR reaction

The expression of Caspase-3 and VEGF$\alpha$ in the anterior cruciate ligament tissue of each group was detected by RT-qPCR.

Primer design

| Caspase-3 | Upstream primer | 5'-GAGCTTGGAACGGTACGATA-3' | SEQ ID NO: 1 |
| | Downstream primer | 5'-CCGTACCAGAGCGAGATGAC-3' | SEQ ID NO: 2 |
| VEGF$\alpha$ | Upstream primer | 5'-GGAGTACCCTGATGAGATCGA -3' | SEQ ID NO: 3 |

(continued)

| | | |
|---|---|---|
| Downstream primer | 5'-CTTTGGTCTGCATTCACATTTGT-3' | SEQ ID NO: 4 |

Caspase-3 plays a key role in apoptosis and is also a potential drug target. Studies have shown that Caspase-3 can severely inhibit DNA replication, transcription, and damage repair functions, ultimately leading to irreversible apoptosis. Therefore, reducing the activation of Caspase-3 can effectively inhibit apoptosis. The results of real-time quantitative PCR for Caspase-3 are shown in Table 4. The results showed that the expression of Caspase-3 mRNA in the control group at any period of time did not change significantly. Compared with the control group, the expression level of Caspase-3 mRNA in the divalent metal lactate group was significantly downregulated on day 1, day 3 and day 7 after modeling ($P < 0.05$). Compared with the magnesium lactate group and the zinc lactate group, the expression level of Caspase-3 mRNA in the magnesium lactate + zinc lactate group was significantly downregulated on day 1, day 3 and day 7 ($P < 0.05$). The above results indicate that the divalent metal lactates can reduce the expression of Caspase-3 protein after ligament tissue injuries in animals and reduce the occurrence of apoptosis caused by nerve injury. The magnesium lactate and zinc lactate combination group showed a synergistic effect in reducing the expression of Caspase-3 protein.

**Table 4: Comparison of Caspase-3 mRNA expression at different time points after modeling and at different time points after surgery**

| Group | Day 1 | Day 3 | Day 7 |
|---|---|---|---|
| Control group | 1.87±0.03 | 1.89±0.16 | 1.82±0.05 |
| Magnesium lactate group | 1.37±0.09 | 1.38±0.25 [a e] | 1.30±0.22 [a f] |
| Magnesium lactate + zinc lactate combination group | 1.01±0.15 [a b d] | 1.12±0.21 [a b d e] | 0.72±0.15 [a b d f] |
| Zinc lactate group | 1.51±0.06 [a b] | 1.53±0.16 [a b e] | 1.61±0.22 [a b f] |
| Note: compared with the control group, [a] $P < 0.05$; compared with the magnesium lactate group, [b] $P < 0.05$; compared with the zinc lactate group, [d] $P < 0.05$; compared day 3 with day 1 in the same group, [e] $P < 0.05$; compared day 7 with day 3 in the same group, [f] $P < 0.05$. | | | |

**Example 3: Effects of the Divalent Metal Lactates on Wound Healing Status in Experimental Animals**

Forty-two SD rats weighing approximately 200 g each (n=6 per group) were anesthetized and then their hair were shaved on their backs. A 5 mm deep and 3 cm long incision was made on each rat's back with a scalpel. 0.5 mL physiological saline was topically applied at the wound for the control group. 25 mmol/L zinc lactate aqueous solution, 50 mmol/L zinc lactate aqueous solution, 25 mmol/L magnesium lactate aqueous solution, 50 mmol/L magnesium lactate aqueous solution, or 25 mmol/L zinc lactate aqueous solution + 25 mmol/L magnesium lactate aqueous solution were topically applied at the wound for each experimental group, respectively. Starting from the day of modeling, each animal was topically applied twice a day in the morning and evening, with 0.5 mL each time, for 14 days. The wound healing rates were recorded on day 3, day 7, and day 14 (see Table 5).

**Table 5: Skin wound healing rates of rats in each group at different time points**

| | Wound healing rate (%) | | |
|---|---|---|---|
| | Day 3 | Day 7 | Day 14 |
| Control group | 32±3.5 | 51±4.4 | 90±3.2 |
| 25 mmol/L zinc lactate aqueous solution group | 37±1.8 [a] | 64±2.7 [b] | 95±1.9 [a] |
| 50 mmol/L zinc lactate aqueous solution group | 52±1.9 [c] | 81±3.9 [c] | 98±3.4 [c] |
| 25 mmol/L magnesium lactate aqueous solution group | 34±2.1 | 61±1.6 [b] | 94±2.8 [a] |
| 50 mmol/L magnesium lactate aqueous solution group | 46±2.3 [c] | 76±1.3 [c] | 97±3.1 [b] |
| 25 mmol/L zinc lactate aqueous solution + 25 mmol/L magnesium lactate aqueous solution group | 61±2.5 [c,e,f] | 93±3.7 [c,e,f] | 99±1.5 [c,d,g] |
| Note: compared with the control group, [a] $P < 0.05$, [b] $P < 0.01$, [c] $P < 0.001$; compared with the 25 mmol/L zinc lactate group, [d] $P < 0.05$, [e] $P < 0.001$; compared with the 25 mmol/L magnesium lactate group, [f] $P < 0.001$, [g] $P < 0.01$. | | | |

The experimental results in Table 5 show that, compared with the animals in the control group, the experimental animals topically applied with 25 mmol/L zinc lactate aqueous solution, 50 mmol/L zinc lactate aqueous solution, 25 mmol/L magnesium lactate aqueous solution, 50 mmol/L magnesium lactate aqueous solution, or 25 mmol/L zinc lactate aqueous solution + 25 mmol/L magnesium lactate aqueous solution had significantly higher wound healing rates at each time point.

As can be seen from the experimental results on day 3 and day 7 in Table 5, although magnesium lactate is not as effective as zinc lactate in promoting skin wound healing, the combination of zinc lactate and magnesium lactate unexpectedly showed a synergistic effect. The wound healing rates of the experimental animals in the combination group on day 3 and day 7 were superior to those in the zinc lactate alone group and the magnesium lactate alone group, respectively.

**Example 4: Effects of the Divalent Metal Lactates on Preventing Wound Scar Formation**

1. Experimental Methods:

Thirty New Zealand big ear white rabbits, both male and female, weighing 1.8-2.2 kg, were used in the experiment. After 7 days of acclimatization feeding, none of them showed any obvious abnormalities.

Establishment of the scar model: Anesthesia was performed by injecting a pentobarbital solution at a concentration of 30 g/L via the ear vein. A circular wound with a diameter of 1 cm was made along the long axis on the ventral side of the rabbit ear. Two wounds were made on each ear, with an interval of about 3.0 cm. The full thickness of the skin was completely excised to form the wound.

Experimental grouping and drug administration: 24 New Zealand big ear white rabbits were randomly divided into 4 groups, and there were 6 animals in each group, with a total of 24 wounds. The groups were a control group, a 50 mmol/L zinc lactate aqueous solution group, a 50 mmol/L magnesium lactate aqueous solution group, and a 25 mmol/L zinc lactate aqueous solution + 25 mmol/L magnesium lactate aqueous solution group, respectively. After the wounds were established, the physiological saline was topically applied at the wounds twice a day in the morning and evening with 0.5 mL each time in the control group. In the experimental group, the corresponding lactate aqueous solution was topically applied at each wound twice a day in the morning and evening with 0.5 mL each time. The evaluation was carried out on day 13.

Therapeutic effect evaluation: the therapeutic effect was evaluated using the Vancouver Scar Scale (VSS) as shown in Table 6. The higher the score, the more severe the scar.

**Table 6: Vancouver Scar Scale scoring criteria**

| Project | Color | Thickness | Blood vessel distribution | Softness |
|---|---|---|---|---|
| 0 point | Skin color is close to normal as compared to the rest of the body | Normal | Normal skin color, close to the rest of the body | Normal |
| 1 point | Lighter color | < 1 mm | Pinkish skin color | Soft (the skin can be deformed with minimal resistance) |
| 2 points | Mixed colors | 1-2 mm | Red skin color | Soft (can be deformed under pressure) |
| 3 points | Deeper color | 2-4 mm | Purple skin color | Hard (cannot be deformed, moves in a blocky shape, and resists to pressure) |
| 4 points | -- | >4 mm | -- | Arcuation (the tissue is rope-like and retracts when the scar is stretched, but joint movement is not affected). |
| 5 points | -- | -- | -- | Contracture (permanent shortening of the scar tissue leads to disability and distortion, and joint movement is limited) |

Statistical analysis: The data were statistically analyzed by using SPSS 21 software. The experimental results are expressed as x±SD.

2. Experimental Results:

The experimental results are shown in Table 7 (Note: the VSS values in Table 7 are the sum of the values of two wounds).

**Table 7: Comparison results of VSS values of local ear scars in New Zealand big ear white rabbits**

| Group | Sample size | Day 13 |
|---|---|---|
| Control group | 24 | $8.39 \pm 1.94$ |
| 50 mmol/L zinc lactate aqueous solution group | 24 | $5.96 \pm 1.66$ |
| 50 mmol/L magnesium lactate aqueous solution group | 24 | $6.84 \pm 1.57$ |
| 25 mmol/L zinc lactate aqueous solution + 25 mmol/L magnesium lactate aqueous solution group | 24 | $3.78 \pm 1.53$ |

The experimental results on day 13 show that, compared with the control group, the VSS scores of the white rabbits in each experimental group decreased to varying degrees, indicating that each experimental group had some effects on preventing wound scar formation. In addition, the VSS scores of the zinc lactate aqueous solution and magnesium lactate aqueous solution combination group were significantly different from those of the zinc lactate group and the magnesium lactate group ($P < 0.01$), indicating that the combination group unexpectedly showed a synergistic effect in preventing wound scar formation.

In addition, in the above applications, the results of other divalent metal lactates, including calcium lactate, zinc lactate, ferrous lactate, and various combinations, were similar to the results of the magnesium lactate described above, and will not be repeatedly described here.

Obviously, those skilled in the art can make various modifications and variations to the present invention without departing from the spirit and scope of the present invention. Thus, if these modifications and variations of the present invention fall within the scope of the claims of the present invention and their equivalents, the present invention also intends to include these modifications and variations.

**Claims**

1.   Use of a divalent metal lactate in the preparation of a product for promoting the regeneration, growth, healing (such as wound healing) and/or injury repair of a tissue, wherein the tissue is selected from collagen-rich tissues.

2.   Use of a divalent metal lactate in the preparation of a product that prevents or treats an injury of a tissue, wherein the tissue is selected from collagen-rich tissues.

3.   Use of a divalent metal lactate in the preparation of a product that prevents or treats the fibrosis and scar generation of a tissue, wherein the tissue is selected from collagen-rich tissues.

4.   The use according to any one of claims 1 to 3, wherein the tissue is selected from at least one of skin, ligaments, tendons (such as achilles tendon) or fasciae.

5.   The use according to any one of claims 1 to 3, wherein the divalent metal lactate comprises one or more of calcium lactate, magnesium lactate, zinc lactate, and ferrous lactate.

6.   The use according to claim 5, wherein the divalent metal lactate is selected from zinc lactate or a composition of zinc lactate and magnesium lactate, preferably, in the composition, the usage ratio of the zinc lactate to the magnesium lactate is 1:10-10:1, preferably 1:1, based on the amount of substance.

7.   The use according to any one of claims 1 to 3, wherein the product is selected from one or more of a drug, a kit, a health product, and a medical device;

preferably, the concentration of the divalent metal lactate in the product is 5-250 mmol/L; more preferably, the content of the divalent metal lactate in the skin, ligaments, tendons or fasciae is 30-150 mmol/L;
preferably, when the product is selected from a drug or a health product, the concentration of the divalent metal lactate in a unit dose of the product is 5-250 mmol/L, preferably 30-150 mmol/L, more preferably 50 mmol/L; more preferably, when the divalent metal lactate is a composition of zinc lactate and magnesium lactate, the sum of the concentrations of the zinc lactate and the magnesium lactate in a unit dose of the product is 5-250 mmol/L, preferably 30-150 mmol/L, more preferably 50 mmol/L; preferably, the concentration of the zinc lactate and the concentration of the magnesium lactate are the same in the composition.

8. The use according to claim 7, wherein the medical device comprises one or more of medical adhesive tape, bandage, gauze, plaster, sponge, and medical sutures.

9. The use according to claim 7, wherein in the drug, the divalent metal lactate is an active ingredient, and the drug further comprises a pharmaceutically acceptable carrier or excipient.

10. The use according to claim 7, wherein the dosage form of the drug comprises an injection or a topical preparation; preferably, the topical preparation comprises one or more of ointments, creams, patches, sprays, solutions, and lotions; preferably, the administration mode of the product comprises one or more of the following: intravenous injection, in situ injection, intramuscular injection, subcutaneous injection, oral administration, or topical application.

**FIG. 1**

**FIG. 2**

**FIG. 3**

## INTERNATIONAL SEARCH REPORT

| International application No. |
| --- |
| **PCT/CN2024/104438** |

| A. | CLASSIFICATION OF SUBJECT MATTER |
| --- | --- |

A61K31/19(2006.01)i; A61K31/185(2006.01)i; A61P17/02(2006.01)i; A61P17/02(2006.01)i; A61P19/04(2006.01)i; A61P41/00(2006.01)i

According to International Patent Classification (IPC) or to both national classification and IPC

| B. | FIELDS SEARCHED |
| --- | --- |

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CJFD, CNTXT, DWPI, ENTXT, ENTXTC, VEN, WPABS, WPABSC, 万方, WANFANG, CNKI, ISI WEB OF SCIENCE, PUB MED, STN: 乳酸, 乳酸钙, 乳酸镁, 乳酸锌, 乳酸亚铁, 乳酸盐, Lactic acid, 50-21-5, 79-33-4, 10326-41-7, Lactate, lactates, Zinc lactate, magnesium lactate, calcium lactate, ferrous lactate, 再生, 愈合, 修复, 胶原, Regeneration, healing, repair, collagen, 纤维化, 瘢痕, 疤, Fibrosis, scar, 皮肤, 韧带, 肌腱, 筋膜, Skin, ligament, tendon, fascia, 软组织, 长春圣博玛生物材料有限公司, 王晋岳, 庄秀丽, 张田慧

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 116459241 A (CHANGCHUN SINOBIOMATERIALS CO., LTD.) 21 July 2023 (2023-07-21) abstract, and description, paragraphs 0002-0024 and 0051-0055 | 7-10 |
| X | US 5981606 A (WARNER-LAMBERT COMPANY) 09 November 1999 (1999-11-09) abstract, and description, column 11, lines 30-64, column 12, lines 25-55, column 16, lines 15-27 and lines 40-52, and column 22, line 14 to column 26, line 18 | 1-10 |
| X | CN 101262871 A (CHIOU CONSULTING INC.) 10 September 2008 (2008-09-10) abstract, and claims 10-12, 15, and 20 | 1-10 |
| X | CN 103316033 A (KANG XIAOFEI) 25 September 2013 (2013-09-25) abstract, claims 3, 7, and 9-10, and description, paragraphs 0029-0031 | 1-10 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **10 October 2024** | **16 October 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**EP 4 744 656 A1**

## INTERNATIONAL SEARCH REPORT

International application No.

**PCT/CN2024/104438**

### C.    DOCUMENTS CONSIDERED TO BE RELEVANT

| Category* | Citation of document, with indication, where appropriate, of the relevant passages | Relevant to claim No. |
|---|---|---|
| X | CN 116077358 A (CHANGCHUN SINOBIOMATERIALS CO., LTD.) 09 May 2023 (2023-05-09) abstract, claims 5, 8, and 10, and description, paragraphs 0033-0036, 0057, and 0103-0124, and embodiment 5 | 1, 2, 4-10 |
| X | US 2018092869 A1 (NELSON, DEANNA J.) 05 April 2018 (2018-04-05) description, paragraphs 0090, 0105, and 0115 | 1-2, 4, 7-10 |
| X | US 5856364 A (WARNER-LAMBERT COMPANY) 05 January 1999 (1999-01-05) abstract, and description, column 10, line 48 to column 11, line 11, column 15, lines 5-38, and column 26, line 30 to column 27, line 8 | 1-10 |
| X | CN 106102733 A (STAYBLE THERAPEUTICS AB) 09 November 2016 (2016-11-09) description, paragraphs 0044-0049, 0057, and 0156-0176 | 1, 2, 4-10 |
| X | US 2005136126 A1 (CAMBRIDGEMED, INC. et al.) 23 June 2005 (2005-06-23) claim 5, and description, paragraph 0012 | 1-10 |

Form PCT/ISA/210 (second sheet) (July 2022)

13

## INTERNATIONAL SEARCH REPORT

| International application No. |
|---|
| **PCT/CN2024/104438** |

| Box No. I      Nucleotide and/or amino acid sequence(s) (Continuation of item 1.c of the first sheet) |
|---|

1. With regard to any nucleotide and/or amino acid sequence disclosed in the international application, the international search was carried out on the basis of a sequence listing:

    a.   ☑ forming part of the international application as filed.

    b.   ☐ furnished subsequent to the international filing date for the purposes of international search (Rule 13*ter*.1(a)),

         ☐ accompanied by a statement to the effect that the sequence listing does not go beyond the disclosure in the international application as filed.

2.   ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2024/104438**

| Patent document cited in search report | | | Publication date (day/month/year) | Patent family member(s) | | | Publication date (day/month/year) |
|---|---|---|---|---|---|---|---|
| CN | 116459241 | A | 21 July 2023 | WO | 2023134146 | A1 | 20 July 2023 |
| | | | | AR | 128163 | A1 | 27 March 2024 |
| | | | | AU | 2022434160 | A1 | 18 July 2024 |
| | | | | TW | 202327561 | A | 16 July 2023 |
| | | | | IL | 314228 | A | 01 September 2024 |
| US | 5981606 | A | 09 November 1999 | None | | | |
| CN | 101262871 | A | 10 September 2008 | JP | 2007513162 | A | 24 May 2007 |
| | | | | US | 2005123620 | A1 | 09 June 2005 |
| | | | | US | 7258875 | B2 | 21 August 2007 |
| | | | | CA | 2539003 | A1 | 23 June 2005 |
| | | | | GB | 0612417 | D0 | 30 August 2006 |
| | | | | GB | 2426454 | A | 29 November 2006 |
| | | | | WO | 2005055927 | A2 | 23 June 2005 |
| | | | | WO | 2005055927 | A3 | 09 November 2006 |
| | | | | TW | 200520762 | A | 01 July 2005 |
| | | | | US | 2008020059 | A1 | 24 January 2008 |
| | | | | KR | 20070000393 | A | 02 January 2007 |
| CN | 103316033 | A | 25 September 2013 | None | | | |
| CN | 116077358 | A | 09 May 2023 | WO | 2023078310 | A1 | 11 May 2023 |
| US | 2018092869 | A1 | 05 April 2018 | US | 10463636 | B2 | 05 November 2019 |
| | | | | WO | 2018084959 | A2 | 11 May 2018 |
| | | | | WO | 2018084959 | A3 | 31 May 2019 |
| | | | | ZA | 201902258 | B | 26 August 2020 |
| | | | | US | 2020016101 | A1 | 16 January 2020 |
| | | | | US | 11026906 | B2 | 08 June 2021 |
| US | 5856364 | A | 05 January 1999 | WO | 9527501 | A1 | 19 October 1995 |
| | | | | EP | 0754052 | A1 | 22 January 1997 |
| | | | | EP | 0754052 | B1 | 11 December 2002 |
| | | | | MX | 9604271 | A | 31 December 1997 |
| | | | | CA | 2184617 | A1 | 19 October 1995 |
| | | | | ES | 2188656 | T3 | 01 July 2003 |
| | | | | DE | 69529158 | D1 | 23 January 2003 |
| | | | | DE | 69529158 | T2 | 21 August 2003 |
| | | | | JPH | 09511746 | A | 25 November 1997 |
| | | | | DK | 0754052 | T3 | 31 March 2003 |
| | | | | NZ | 283934 | A | 26 August 1998 |
| CN | 106102733 | A | 09 November 2016 | JP | 2017508813 | A | 30 March 2017 |
| | | | | JP | 6587675 | B2 | 09 October 2019 |
| | | | | LT | 3119386 | T | 10 October 2019 |
| | | | | PT | 3119386 | T | 01 October 2019 |
| | | | | US | 2018169041 | A1 | 21 June 2018 |
| | | | | ES | 2746075 | T3 | 04 March 2020 |
| | | | | IL | 247882 | A0 | 30 November 2016 |
| | | | | IL | 247882 | B | 31 October 2019 |
| | | | | RU | 2016137165 | A | 26 April 2018 |
| | | | | RU | 2016137165 | A3 | 17 September 2018 |
| | | | | RU | 2682675 | C2 | 20 March 2019 |
| | | | | KR | 20160125518 | A | 31 October 2016 |
| | | | | KR | 102329185 | B1 | 19 November 2021 |

Form PCT/ISA/210 (patent family annex) (July 2022)

**INTERNATIONAL SEARCH REPORT**
Information on patent family members

International application No.

**PCT/CN2024/104438**

| Patent document cited in search report | | | Publication date (day/month/year) | Patent family member(s) | | | Publication date (day/month/year) |
|---|---|---|---|---|---|---|---|
| | | | | CY | 1122042 | T1 | 14 October 2020 |
| | | | | PL | 3119386 | T3 | 31 December 2019 |
| | | | | DK | 3119386 | T3 | 16 September 2019 |
| | | | | HRP | 20191633 | T1 | 13 December 2019 |
| | | | | AU | 2015233396 | A1 | 29 September 2016 |
| | | | | AU | 2015233396 | B2 | 30 January 2020 |
| | | | | MX | 2016012050 | A | 13 April 2017 |
| | | | | EP | 3119386 | A1 | 25 January 2017 |
| | | | | EP | 3119386 | B1 | 19 June 2019 |
| | | | | SI | 3119386 | T1 | 29 November 2019 |
| | | | | CA | 2943256 | A1 | 24 September 2015 |
| | | | | CA | 2943256 | C | 27 April 2021 |
| | | | | WO | 2015140320 | A1 | 24 September 2015 |
| | | | | HUE | 046209 | T2 | 28 February 2020 |
| | | | | RS | 59285 | B1 | 31 October 2019 |
| | | | | US | 2018280327 | A1 | 04 October 2018 |
| | | | | US | 10322098 | B2 | 18 June 2019 |
| US | 2005136126 | A1 | 23 June 2005 | WO | 2006074221 | A2 | 13 July 2006 |
| | | | | WO | 2006074221 | A3 | 28 September 2006 |

Form PCT/ISA/210 (patent family annex) (July 2022)

**REFERENCES CITED IN THE DESCRIPTION**

**Patent documents cited in the description**

- CN 202310840152

- CN 202210028046